# EUROPEAN PATENT APPLICATION

(11) **EP 4 745 244 A1**
(43) Date of publication of application: **20.05.2026**
(21) Application number: 23944733.7
(22) Date of filing: 13.07.2023
(51) Int. Cl.: C12P 7/64, C12N 15/81, C12N 15/65, C12R 1/865, C12P 5/02

(54) **ENGINEERING BIOLOGICAL CONTAINMENT SYSTEM AND METHOD**

(71) Applicant: BGI Shenzhen, Shenzhen, Guangdong 518083 (CN)
(72) Inventor: FU, Xian, Shenzhen, Guangdong 518083 (CN); DING, Weichao, Shenzhen, Guangdong 518083 (CN); YAN, Shirui, Shenzhen, Guangdong 518083 (CN); CHANG, Tiantian, Shenzhen, Guangdong 518083 (CN); SHEN, Yue, Shenzhen, Guangdong 518083 (CN)
(74) Representative: Schwarz & Partner Patentanwälte GmbH
(86) International application number: PCT/CN2023/107221
(87) International publication number: WO 2025/010715

(57) **Abstract**

An engineered biological containment system, comprising: a first nucleic acid, the first nucleic acid comprising at least a fragment portion of a first essential gene; a second nucleic acid, the second nucleic acid comprising at least a fragment portion of a second essential gene; a third nucleic acid, the third nucleic acid activating the transcription of the first nucleic acid; and a fourth nucleic acid, the fourth nucleic acid encoding a non-natural amino acid encoding tool. The second nucleic acid comprises one or more nucleotide sequences encoding a first termination codon and optionally a nucleotide sequence located at the end position of a second-essential-gene encoding region and encoding a second termination codon.

## Description

### FIELD

The present disclosure relates to the technical field of bioengineering, and specifically to an engineered biological containment system and method, an engineered organism obtained thereto, and use of the engineered organism.

### BACKGROUND

The rapid development of engineered organisms and synthetic biology has greatly advanced the progress and application of biotechnology. At present, artificially genetically modified organisms have demonstrated significant application prospects in industrial production, clinical medicine, environmental remediation and other fields. For example, as a landmark achievement in artificially synthesized organisms, scientists have obtained *Saccharomyces cerevisiae* containing multiple de novo designed and synthesized chromosomes, which can be used to develop high-yield strains with optimized compatibility between exogenous metabolic pathways and chassis strains. However, while these engineered organisms inject vitality into the development of scientific research and industry, they also bring about problems such as the leakage of genetic information of artificially genetically modified organisms. These potential safety issues may exert negative impacts on the natural environment, ecology and human health.

To prevent engineered organisms from escaping into the external environment, containment strategies in the related art include auxotrophic biological containment, inducible biological containment and gene flow barrier. The auxotrophic biological containment usually knocks out the genes required for synthesizing essential substances in engineered organisms, such that the growth of engineered organisms is dependent on exogenously supplied nutrients. Once engineered organisms escape, they will fail to grow due to the lack of corresponding nutrients in the environment. Nevertheless, since various metabolically essential molecules are widely present in the natural environment, such auxotrophic strains can survive continuously, rendering the biological containment strategy ineffective. The inducible biological containment not only requires extensive experimental verification, but also cannot prevent engineered organisms from escaping into the external environment through altering their own metabolic pathways and horizontal gene transfer. Moreover, the gene flow barrier achieves biological containment through toxin-antitoxin systems, so when gene mutations occur in toxin or antitoxin proteins, such a biological containment may become invalid.

Therefore, there is an urgent need to develop simple, effective and widely applicable biological containment methods to improve human control over genetically modified organisms.

### SUMMARY

For this purpose, in a first aspect, the present disclosure provides, in embodiments, an engineered biological containment system, including: a first nucleic acid, including at least a partial sequence of a first essential gene; a second nucleic acid, including at least a partial sequence of a second essential gene; a third nucleic acid activating the transcription of the first nucleic acid; and a fourth nucleic acid, encoding an unnatural-amino-acid encoding tool; wherein the second nucleic acid contains one or more nucleotide sequence(s) encoding a first termination codon(s), and optionally contains a nucleotide sequence encoding a second termination codon located at the end of a coding region of the second essential gene.

In some embodiments, the first essential gene is the same as the second essential gene. In other embodiments, the first essential gene is different from the second essential gene.

In some embodiments, the third nucleic acid is an inducible promoter, preferably an inducible promoter pGAL and/or CaMV35S.

In some embodiments, the first termination codon encodes an unnatural amino acid.

In some embodiments, the nucleotide sequence encoding the first termination codon replaces a nucleotide sequence encoding one or more amino acids among the first 20 amino acids at an N-terminus of a protein encoded by the second essential gene. In some embodiments, the nucleotide sequence encoding the first termination codon replaces a nucleotide sequence encoding one or more amino acids among the 4th to the 20th amino acids at the N-terminus of the protein encoded by the second essential gene.

In some embodiments, the second essential gene includes a gene with a medium-to-low expression level, optionally, the second essential gene includes at least one of *CDC27* and *CDC4.*

In some embodiments, the nucleotide sequence encoding the first termination codon replaces a nucleotide sequence encoding any one or a combination of the following amino acids of *CDC4:* F4, P5, L6, A7, E8, F9, P10, L11, R12, D13, I14, P15, V16, P17, Y18, S19 and Y20; and/or the nucleotide sequence encoding the first termination codon replaces a nucleotide sequence encoding any one or a combination of the following amino acids of *CDC27:* N4, P5, E6, L7, A8, P9, F10, T11, L12, S13, R14, G15, I16, P17, S18, F19 and D20.

In some embodiments, the nucleotide sequence encoding the first termination codon replaces a nucleotide sequence encoding any one or a combination of the following amino acids of *CDC4:* F9, R12 and S19; and the nucleotide sequence encoding the first termination codon replaces a nucleotide sequence encoding any one or a combination of the following amino acids of *CDC27:* P5, L7, A8, T11, R14, P17, F9 and D20.

In some embodiments, the second essential gene includes at least one of *CDC27* and *CDC4.*

In some embodiments, the nucleotide sequence encoding the first termination codon replaces a nucleotide sequence encoding the 520th amino acid of *CDC27*; and/or the nucleotide sequence encoding the first termination codon replaces a nucleotide sequence encoding the 325th amino acid of *CDC4.*

In some embodiments, the unnatural-amino-acid encoding tool includes a methoxytyrosyl-tRNA synthetase/leucine tRNA orthogonal pair (LeuOmeRS/tRNA_{CUA}), and the unnatural amino acid encoded by the first termination codon includes O-methyl-L-tyrosine (OmeY).

In some embodiments, the first essential gene is selected from the group consisting of *RPC11, SKP1* and *RPS3.*

In some embodiments, the engineered biological containment system further includes a vector selected from the group consisting of a eukaryotic cell expression vector and a prokaryotic cell expression vector. Optionally, the eukaryotic cell expression vector includes pXF412, pXF413, pXF414, pXF415, pXF294, pXF259, pDR196, pHISi, pESP-3, pESP-2, pESP-1, pHiSi-1, pGAG424, p53his, pRS426gal, pRS41H, pRS413, pRS416, pGBT9 and pAUR123; optionally, the prokaryotic cell expression vector includes pET28a-TagRFP-N, pTrc-CKS, pET-DsbA, pET-Trx, pET-28a(+)-GFP, pET-28a(+)-sumo, pET-3c-sumo, pET-35b(+), pTXB1, and pCWori.

In some embodiments, the vector includes: a first vector including the second nucleic acid, and optionally the first nucleic acid; a second vector including the third nucleic acid; and a third vector including the fourth nucleic acid.

In some embodiments, the vector further includes a replication origin, a selection marker and a restriction site. Optionally, the replication origin is at least one of ARS and Ori. Optionally, the selection marker is a nucleotide sequence encoding at least one of the following proteins: His, Ura, Leu, Amp and Kan. Optionally, the restriction site is at least one of the following: ApaI, BamHI, BglII, EcoRI, HindIII, KpnI, NcoI, NdeI, NheI, NotI, SacI, SalI, SphI, XbaI and XhoI.

In a second aspect, the present disclosure provides, in embodiments, an engineered biological containment method, including: introducing a third nucleic acid and a fourth nucleic acid into an organism to be engineered, wherein the third nucleic acid activates the transcription of a first essential gene of the organism to be engineered, and the fourth nucleic acid encodes an unnatural-amino-acid encoding tool; and including one or more nucleotide sequence(s) encoding a first termination codon(s) in a second essential gene of the organism to be engineered, optionally including a nucleotide sequence encoding a second termination codon at the end of a coding region of the second essential gene.

In some embodiments, the first essential gene is the same as the second essential gene. In other embodiments, the first essential gene is different from the second essential gene.

In some embodiments, the third nucleic acid is an inducible promoter, preferably an inducible promoter pGAL and/or CaMV35S. In some embodiments, the first termination codon encodes an unnatural amino acid.

In a third aspect, the present disclosure provides, in embodiments, an engineered organism, containing an engineered biological containment system in any one of embodiments in the first aspect of the present disclosure.

In some embodiments, the engineered organism includes an eukaryotic cell and a prokaryote, wherein the eukaryotic cell includes: a yeast cell, an animal cell including a mammalian cell and an insect cell, and a plant cell including a tobacco cell, an *Arabidopsis* cell and a *Columnea* cell; and wherein the prokaryote includes *Escherichia coli* and *Bacillus subtilis.*

In a fourth aspect, the present disclosure provides, in embodiments, use of an engineered organism in any one of embodiments in the third aspect of the present disclosure in the production of sustainable biomass including food, feed, pharmaceutical, biofuel and material.

In a fifth aspect, the present disclosure provides, in embodiments, a method for screening an engineered biological containment system, including: replacing, with a nucleotide sequence encoding a first termination codon, a nucleotide sequence encoding one or more amino acids among the first 20 amino acids at the N-terminus of a protein encoded by a second essential gene of an engineered organism, so as to obtain a mutant library of engineered organisms, where the first termination codon encodes an unnatural amino acid; introducing a fourth nucleic acid encoding an unnatural-amino-acid encoding tool into the mutant library of engineered organisms; and screening the mutant library of engineered organisms based on a first culture condition, so as to obtain the engineered biological containment system.

In some embodiments, the first culture condition is the presence of the unnatural amino acid.

In some embodiments, the method for screening an engineered biological containment system further includes: introducing a third nucleic acid activating the transcription of a first essential gene of the engineered organism into the mutant library of engineered organisms. Optionally, the third nucleic acid is an inducible promoter, preferably an inducible promoter pGAL and/or CaMV35S. Optionally, the first essential gene is the same as or different from the second essential gene.

In some embodiments, the method for screening an engineered biological containment system further includes: determining a second culture condition according to the inducible promoter; and screening the mutant library of engineered organisms based on the first culture condition and the second culture condition, so as to obtain the engineered biological containment system.

In some embodiments, the second culture condition is the presence of Gal and Glu (i.e., providing Gal and Glu).

In some embodiments, the method for screening an engineered biological containment system further includes: replacing, with the nucleotide sequence encoding the first termination codon, a nucleotide sequence encoding one or more amino acids among the 4th to the 20th amino acids at the N-terminus of the protein encoded by the second essential gene of the engineered organism, so as to obtain a mutant library of engineered organisms.

The solution for biological containment provided in the present disclosure has the following beneficial technical effects.
i. It greatly reduces the possibility of the escape of engineered yeast, with an escape rate lower than the assay limit of the detection method (10⁻⁹).
ii. The constructed N-terminal library of the target protein encompasses every residue at the N-terminus of the target essential protein, such that through subsequent analysis of growth curve and sequencing, the essential genes and specific substitution sites can be rapidly identified without affecting the growth of the strain.
iii. It is of universality, being applicable to most intracellular essential genes and suitable for *Saccharomyces cerevisiae* and other organisms.

### BRIEF DESCRIPTION OF THE DRAWINGS

A brief description will be made to the accompanying drawings as required in embodiments, to illustrate the technical solutions in the embodiments of the disclosure more clearly. Apparently, the accompanying drawings set forth in the following description are some examples of this disclosure. For those of ordinary skill in the art, other accompanying drawings could also be obtained based on these drawings without exerting creative efforts.

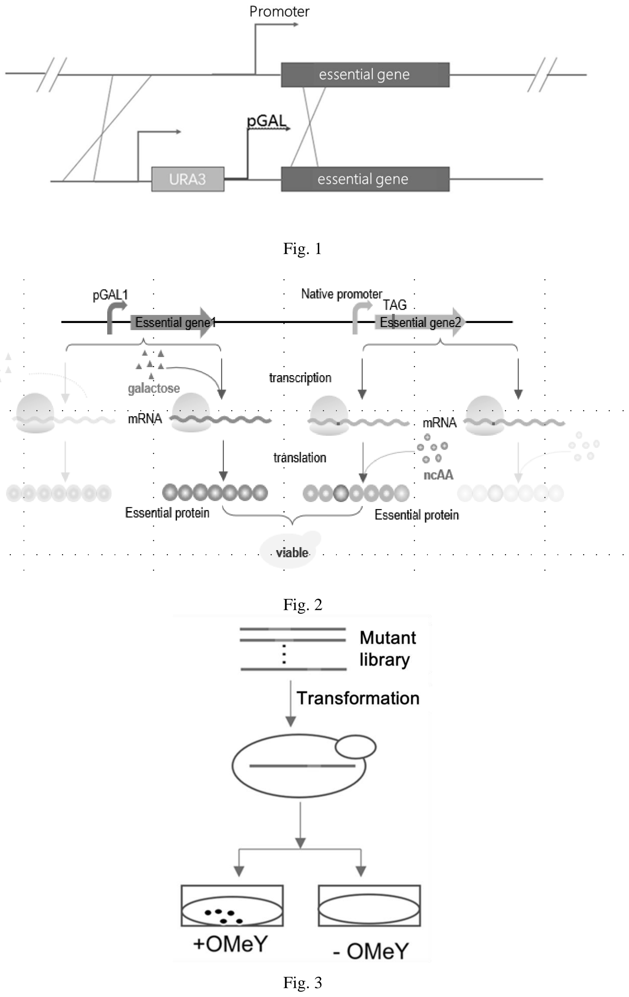

Fig. 1 is a schematic diagram illustrating a replacement with a promoter pGAL according to embodiments of the present disclosure.
Fig. 2 is a schematic diagram illustrating the principle of a biological containment method with transcription-translation co-regulation according to embodiments of the present disclosure.
Fig. 3 is a flow diagram showing a screening on sites in an N-terminal library of a target protein according to embodiments of the present disclosure.
Fig. 4 is a schematic diagram illustrating a genome recombination of a transcriptionally regulated strain according to embodiments of the present disclosure.
Fig. 5 shows the results of gradient dilution spot assay of strains yXF239, yXF240, yXF241 and WT according to embodiments of the present disclosure.
Fig. 6 shows the results of growth activity determination for transcription-regulated strains according to embodiments of the present disclosure.
Fig. 7 shows the results of growth activity determination for transcription-translation co-regulated strains according to embodiments of the present disclosure.
Fig. 8 shows escape rates in a verification on transcriptional-translational co-regulation according to embodiments of the present disclosure.
Fig. 9 shows a gel electrophoresis image of PCR products of the gene sequences of *CDC27* and *CDC4* according to embodiments of the present disclosure.
Fig. 10 is a flow diagram showing a construction of an N-terminal library of a target protein and a screening thereon according to embodiments of the present disclosure.
Fig. 11 shows the proportional distribution of CDC4 mutant library after transformation according to embodiments of the present disclosure.
Fig. 12 shows the proportional distribution of CDC27 mutant library after transformation according to embodiments of the present disclosure.
Fig. 13 shows doubling times of strains with different substitution sites in *CDC4* according to embodiments of the present disclosure.
Fig. 14 shows doubling times of strains with different substitution sites in *CDC27* according to embodiments of the present disclosure.

### DETAILED DESCRIPTION

Reference will be made in details further to the disclosure in combination with the specific embodiments. The embodiments described herein are used to illustrate the present disclosure, but not to limit the scope thereof. The embodiments set forth below may serve as a guide for those of ordinary skill in the art to make further improvements, and shall not be construed to limit the present disclosure in any form.

The present disclosure is made based on the inventors' findings as follows.

In the related art, biological containment strategies for engineered organisms suffer from prominent problems of failure caused by gene mutations, supplementation of metabolites from the natural environment, and horizontal gene transfer. The efficiency of previous biological containment methods is far from satisfactory: the escape rate of engineered organisms under non-permissive conditions is higher than 10⁻⁸, while under permissive conditions, the growth activity of engineered organisms is also significantly reduced due to genetic engineering modification. In addition, the selection of insertion sites for codons encoding unnatural amino acids requires sophisticated and elaborate design as well as extensive experiments, which entails high time and labor costs.

Based on this, the present disclosure provides, in embodiments, an engineered biological containment system, including: a first nucleic acid, including at least a partial sequence of a first essential gene; a second nucleic acid, including at least a partial sequence of a second essential gene; a third nucleic acid activating the transcription of the first nucleic acid; and a fourth nucleic acid encoding an unnatural-amino-acid encoding tool; where the second nucleic acid contains one or more nucleotide sequence(s) encoding a first termination codon(s), and optionally contains a nucleotide sequence encoding a second termination codon located at the end of a coding region of the second essential gene.

In some embodiments, the first nucleic acid includes at least a partial sequence of the first essential gene, and the third nucleic acid is an inducible promoter capable of activating the transcription of the first nucleic acid under a first permissive condition. The second nucleic acid includes at least a partial sequence of the second essential gene and one or more nucleotide sequence(s) encoding the first termination codon(s), while the fourth nucleic acid encodes an unnatural-amino-acid encoding tool, by which, under a second permissive condition, the fourth nucleic acid could incorporate the unnatural amino acid into the translation process of the second essential gene, enabling a complete synthesis of the protein encoded by the second essential gene in the organism.

In embodiments of the present disclosure, the term "essential genes" refers to genes that cause a lethal phenotype when deleted. Essential genes are genes that play a decisive role in the survival and development of a species under certain conditions. It is to be understood that although different species have different genome sizes and gene compositions, these highly divergent genomes all contain their own set of essential genes to maintain key cellular functions. Essential genes reveal that the most fundamental biological mechanisms of a species are the necessary conditions for the survival of that species. Essential genes vary among different species; for example, in yeast, essential genes include *CDC27, CDC4, MET30, RPC11, SKP1, RPS3,* and others.

In some embodiments, the first essential gene and the second essential gene may be the same or different. It is to be understood that when the first essential gene is identical to the second essential gene, containment at two levels, i.e. the transcriptional and translational levels, is imposed on the same essential gene. Such simultaneous containment at both the transcriptional and translational levels is achieved by controlling the expression of this essential gene with an introduced exogenous promoter, and replacing a nucleotide sequence encoding a certain amino acid in the coding region of this essential gene with a codon for an unnatural amino acid. In some embodiments, the upstream region of the second essential gene contains a promoter that directly regulates the transcription of the essential gene, and in this case, the first essential gene may be the same as the second essential gene. In some embodiments, the upstream region of the first essential gene contains a promoter, while the upstream region of the second essential gene does not contain a promoter, and in this case, the first essential gene and the second essential gene may be different genes.

In some embodiments, the first essential gene is selected from the group consisting of *CDC27, CDC4, MET30, RPC11, SKP1,* and *RPS3.* In some embodiments, the first essential gene is selected from the group consisting of *RPC11, SKP1,* and *RPS3.* In some embodiments, the first essential gene is *RPS3.*

In some embodiments, the second essential gene includes at least one of *CDC27, CDC4, MET30, RPC11, SKP1,* and *RPS3.* In some embodiments, the second essential gene includes at least one of *CDC27* and *CDC4.* In some embodiments, the nucleotide sequence encoding the first termination codon replaces a nucleotide sequence encoding the 520th amino acid of *CDC27;* and/or the nucleotide sequence encoding the first termination codon replaces a nucleotide sequence encoding the 325th amino acid of *CDC4.*

In the embodiments of the present application, the "first nucleic acid" and the "second nucleic acid" include at least partial sequences of the first and second essential genes, respectively. It is to be understood that the "at least partial sequence" refers to any partial fragment of the essential gene. In some embodiments, the "at least partial sequence" is the full-length sequence of the essential gene. In some embodiments, the "at least partial sequence" is the coding region sequence of the essential gene.

In embodiments of the present disclosure, the third nucleic acid is an inducible promoter. An "inducible promoter" refers to a promoter that can activate or significantly enhance the transcriptional level of a gene in response to certain specific physical or chemical signals. It includes natural inducible promoters such as photo-inducible gene promoters, heat-inducible gene promoters, wound-inducible gene promoters, fungal-inducible gene promoters, symbiotic bacteria-inducible gene promoters, and the like; as well as artificially constructed inducible promoters such as a pGAL promoter, CaMV35S and the like. The full name of the pGAL promoter is galactokinase gene (*GAL1*) promoter, which is an inducible promoter in *Saccharomyces cerevisiae,* and its expression efficiency is regulated by galactose (Gal) and glucose (Glu). Under culture conditions rich in Glu, this promoter is turned off, whereas under culture conditions lacking Glu but containing Gal, this promoter is activated. In this model, Gal and Glu act as functional elements for promoter activation and turn-off, respectively, thereby turning on and off the expression of downstream genes of the promoter. Considering this characteristic of such a promoter, the method provided in embodiments of the present disclosure applies the promoter to an essential gene, thereby specifically controlling the expression and turn-off of the essential gene, which in turn regulates cell survival and death, achieving the purpose of biological containment to prevent escape.

It is to be understood that the first permissive condition depends on the inducible promoter. In some embodiments, the inducible promoter is an pGAL inducible promoter, and the first permissive condition is the presence of only galactose(Gal) in a culture medium, under which the promoter is activated, thereby initiating the expression of the first essential gene; while the non-permissive condition is the presence of glucose(Glu) in the culture medium, under which the transcription of the first essential gene cannot be activated, thereby inhibiting the growth of the engineered organism.

In embodiments of the present disclosure, the nucleotide sequence encoding the first termination codon replaces a nucleotide sequence encoding one or more amino acids among the first 20 amino acids at the N-terminus of a protein encoded by the second essential gene. In some embodiments, the nucleotide sequence encoding the first termination codon replaces a nucleotide sequence encoding one or more amino acids among the 4th to the 20th amino acids at the N-terminus of the protein encoded by the second essential gene. The inventors have found that modifying the beginning region spanning the 4th to the 20th amino acids at the N-terminal of most essential proteins does not affect the structure and function of the essential proteins. Thus, the biological containment system proposed in embodiments of the present disclosure can effectively ensure the normal viability of the engineered organism, allowing it to survive and perform production functions normally.

In some embodiments, the nucleotide sequence encoding the first termination codon replaces a nucleotide sequence encoding any one or a combination of the following amino acids of *CDC4:* F4, P5, L6, A7, E8, F9, P10, L11, R12, D13, I14, P15, V16, P17, Y18, S19 and Y20; and/or the nucleotide sequence encoding the first termination codon replaces a nucleotide sequence encoding any one or a combination of the following amino acids of *CDC27:* N4, P5, E6, L7, A8, P9, F10, T11, L12, S13, R14, G15, 116, P17, S18, F19 and D20.

In embodiments of the present disclosure, the first termination codon encodes an unnatural amino acid. Since unnatural amino acids do not exist in natural conditions, the second essential gene cannot be completely translated. In the presence of the unnatural amino acid, the unnatural-amino-acid encoding tool can incorporate the unnatural amino acid into the translation process of the second essential gene, allowing it to synthesize a protein normally, thereby achieving the purpose of biological containment at the translational level.

In embodiments of the present disclosure, the term "second termination codon" encodes a naturally occurring stop codon located at the 3' end of the mRNA transcribed from the coding region sequence of the essential gene. The "first termination codon" refers to a stop codon located at a position within the essential gene excluding the 3' end of the mRNA at which the naturally occurring "second termination codon" located. In some embodiments, the first termination codon and the second termination each include UAG, UAA, and UGA. The nucleotide sequences encoding the first termination codon and the second termination codon each include TAG, TAA, and TGA. In some embodiments, the first termination codon is UGA, and the nucleotide sequence encoding the first termination codon is TAG.

The term "unnatural amino acid" refers to an amino acid that is not encoded by the existing 64 genetic codons. In embodiments of the present disclosure, the unnatural amino acid includes O-methyl-L-tyrosine (OmeY).

In embodiments of the present disclosure, the fourth nucleic acid encodes the above unnatural-amino-acid encoding tool. The unnatural-amino-acid encoding tool includes a tRNA synthetase (RS) and a tRNA specific to a certain type of unnatural amino acid. The tRNA synthetase in this tool can specifically recognize the target unnatural amino acid and catalyze the loading of the unnatural amino acid onto the tRNA, thereby activating the tRNA, and the activated tRNA can specifically recognize the codon encoding the unnatural amino acid. In embodiments of the present disclosure, the unnatural-amino-acid encoding tool includes a methoxytyrosyl-tRNA synthetase/leucine tRNA orthogonal pair (LeuOmeRS/tRNA_{CUA}).

It is to be understood that the second permissive condition depends on the unnatural amino acid encoded by the termination codon. In some embodiments, the termination codon is the amber codon (TAG), and the second permissive condition is the introduction of an exogenous unnatural amino acid, in which case the unnatural-amino-acid encoding tool is, for example, the methoxytyrosyl-tRNA synthetase/leucine tRNA orthogonal pair (LeuOmeRS/tRNA_{CUA}). As an unnatural-amino-acid encoding tool, LeuOmeRS in this tool can specifically recognize the unnatural amino acid O-methyl-L-tyrosine (OmeY) and tRNA_{CUA}, and catalyze the loading of OmeY onto the 3'-CCA of tRNA_{CUA} to activate tRNA_{CUA}. The activated tRNA_{CUA} reads through the termination codon to produce a complete-length essential protein, allowing the organism to survive. Since unnatural amino acids are absent in the natural environment, escaped strains cannot produce the full-length essential protein and can only survive when the unnatural amino acid is artificially supplied, thereby reducing the possibility of escape of the engineered organism.

In some embodiments, the system further includes a vector selected from the group consisting of eukaryotic cell expression vectors and prokaryotic cell expression vectors.

In embodiments of the present disclosure, the term "vector" refers to a nucleic acid molecule, preferably an artificial nucleic acid molecule. Vectors in the context of embodiments of the present disclosure are suitable for incorporating or carrying desired nucleic acid sequences, such as nucleic acid sequences containing open reading frames. Such vectors may be storage vectors, expression vectors, cloning vectors, transfer vectors, etc. A storage vector is a vector that allows convenient storage of nucleic acid molecules (e.g., mRNA molecules). Accordingly, a vector may contain a sequence corresponding to, for example, a desired mRNA sequence or a portion thereof, such as a sequence corresponding to the open reading frame and 3'-UTR of the mRNA. An expression vector can be used to produce expression products such as RNA (e.g., mRNA) or peptides, polypeptides, or proteins. For instance, an expression vector may contain sequences required for transcribing a vector sequence fragment, such as a promoter sequence (e.g., an RNA polymerase promoter sequence). A cloning vector is usually a vector containing a cloning site that can be used to incorporate a nucleic acid sequence into the vector. Cloning vectors may be, for example, plasmid vectors or phage vectors. A transfer vector may be a vector suitable for transferring nucleic acid molecules into cells or organisms, such as viral vectors. Vectors in the context of embodiments of the present disclosure may be, for example, eukaryotic cell expression vectors or prokaryotic cell expression vectors. In some embodiments, eukaryotic cell expression vectors include pDR196, pHISi, pESP-3, pESP-2, pESP-1, pHiSi-1, pGAG424, p53his, pRS426gal, pRS41H, pRS413, pRS416, pGBT9 and pAUR123; prokaryotic cell expression vectors include pET28a-TagRFP-N, pTrc-CKS, pET-DsbA, pET-Trx, pET-28a(+)-GFP, pET-28a(+)-sumo, pET-3c-sumo, pET-35b(+), pTXB1 and pCWori. Preferably, the vector is a eukaryotic vector, such as pRS413 or pRS416. Preferably, a vector in the sense of the present disclosure contains a restriction sites, a selection marker, and sequences suitable for vector propagation, such as a replication origin. Preferably, the vector in the context of embodiments of the present disclosure is a plasmid vector. Optionally, the replication origin is at least one of ARS and Ori. Optionally, the selection marker is a nucleotide sequence encoding at least one of the following proteins: His, Ura, Leu, Amp, and Kan. Optionally, the restriction site is at least one of the following: ApaI, BamHI, BglII, EcoRI, HindIII, KpnI, NcoI, NdeI, NheI, NotI, SacI, SalI, SphI, XbaI and XhoI.

In some embodiments, the vector includes: a first vector including the second nucleic acid; a second vector including the third nucleic acid; and a third vector including the fourth nucleic acid.

In some embodiments, the first vector includes the second nucleic acid and the first nucleic acid.

In summary, the first permissive condition and the second permissive condition can only be satisfied simultaneously when both an inducer of the inducible promoter and the unnatural amino acid are present. In contrast, the common conditions in nature are non-permissive conditions, which are of the absence of Gal/presence of Glu and natural amino acids. Under such non-permissive conditions, engineered organisms equipped with the biological containment system in embodiments of the present disclosure cannot grow, thereby realizing a safe, efficient, easy-to-operate, and widely applicable biological containment.

In a second aspect, the present disclosure provides, in embodiments, an engineered biological containment method, including: introducing a third nucleic acid and a fourth nucleic acid into an organism to be engineered, where the third nucleic acid activates the transcription of a first essential gene of the organism to be engineered, and the fourth nucleic acid encodes an unnatural-amino-acid encoding tool; and including one or more nucleotide sequence(s) encoding a first termination codon(s) in a second essential gene of the organism to be engineered, and optionally including a nucleotide sequence encoding a second termination codon at the end of a coding region of the second essential gene. The engineered biological containment method in embodiments of the present disclosure can realize a safe, efficient, easy-to-operate, and widely applicable biological containment.

In a third aspect, the present disclosure provides, in embodiments, an engineered organism, containing an engineered biological containment system in any one of embodiments in the first aspect of the present disclosure.

In embodiments of the present disclosure, the term "engineered organism" refers to an engineered strain or engineered cell line that is directionally modified and cultured on a large scale to produce large quantities of useful metabolites or exert its unique physiological functions. It is to be understood that the unnatural-amino-acid encoding system and biological containment strategy proposed in the present disclosure have universality and any chassis strain used in production could be modified with this system and strategy to prevent biological escape of the strain. The present application is not intended to limit the specific category of the engineered organism.

In some embodiments, the engineered organism includes an eukaryotic cell and a prokaryote, where the eukaryotic cell includes: a yeast cell, an animal cell including a mammalian cell and an insect cell, and a plant cell including a tobacco cell, an *Arabidopsis* cell and a *Columnea* cell; and where the prokaryote includes *Escherichia coli* and *Bacillus subtilis.*

In a fourth aspect, the present disclosure provides, in embodiments, use of an engineered organism in any one of embodiments in the third aspect of the present disclosure in the production of sustainable biomass including food, feed, pharmaceutical, biofuel and material.

In a fifth aspect, the present disclosure provides, in embodiments, a method for screening an engineered biological containment system, including: replacing, with a nucleotide sequence encoding a first termination codon, a nucleotide sequence encoding one or more amino acids among the first 20 amino acids at the N-terminus of a protein encoded by a second essential gene of an engineered organism, so as to obtain a mutant library of engineered organisms, where the first termination codon encodes an unnatural amino acid; introducing a fourth nucleic acid encoding an unnatural-amino-acid encoding tool into the mutant library of engineered organisms; and screening the mutant library of engineered organisms based on a first culture condition, so as to obtain the engineered biological containment system.

In some embodiments, the method further includes: introducing a third nucleic acid activating the transcription of a first essential gene of the engineered organism into the mutant library of engineered organisms.

In some embodiments, the method further includes: determining a second culture condition according to the inducible promoter; and screening the mutant library of engineered organisms based on the first culture condition and the second culture condition, so as to obtain the engineered biological containment system.

It should be explained that the screening on the mutant library of the engineered organism is performed by verifying the growth activity and escape rate of the engineered organism. Those skilled in the art will appreciate that the doubling time of cells can be used to reflect the growth activity of the strain: strains with high growth activity have a shorter doubling time, whereas those with low growth activity have a prolonged doubling time. The doubling time of the strain can be accurately calculated by continuous culture and continuous detection of biomass in the culture system, thereby quantifying the growth activity of the strain.

Those skilled in the art will understand that the escape rate can be used to evaluate the biological containment effect. There is a correlation between the volume of the microbial culture and the inoculation amount. When cells in the solution are plated under permissive conditions, all cells can grow into colonies on the plate; whereas cells that grow under non-permissive conditions have evaded the biological containment. Therefore, the escape rate can be calculated by using the number of colonies grown on plates inoculated with a large volume of microbial culture under non-permissive conditions as the denominator, and the number of colonies grown on plates inoculated with a quantified volume of microbial culture under permissive conditions as the numerator. In some specific embodiments, the escape rate is calculated as follows: after inoculating m mL of microbial culture onto SC solid medium plates and culturing for 2 days, the number of single colonies grown is x; the microbial culture is serially diluted and inoculated onto SC-Leu-Ura solid medium plates to determine the minimum inoculation amount n that allows colony growth, with the corresponding number of single colonies being y, and the escape rate of the strain is calculated by the formula: Escape Rate = (y × n) / (x × m).

Since the screening on the mutant library of the engineered organism is based on the verification of growth activity and escape rate of the engineered organism, and it is needed to record the respective number of single colonies under non-permissive and permissive conditions for calculating the escape rate. Accordingly, in some embodiments, the first culture condition includes two types of media: one supplemented with the unnatural amino acid and the other without the unnatural amino acid. In some embodiments, the second culture condition includes two types of media: one supplemented with Gal and the other supplemented with Glu.

In some embodiments, the method further includes: replacing, with the nucleotide sequence encoding the first termination codon, a nucleotide sequence encoding one or more amino acids among the 4th to the 20th amino acids at the N-terminus of the protein encoded by the second essential gene of the engineered organism, so as to obtain a mutant library of engineered organisms.

The screening method for an engineered biological containment system provided in embodiments of the present disclosure can rapidly identify suitable insertion sites for the unnatural amino acid, thereby saving substantial time and labor costs.

The engineered organism provided in embodiments of the present disclosure is of high controllability due to modifications for containing the unnatural amino acid encoding system described in the above embodiments. Accordingly, by controlling the engineered organism-i.e., implementing biological containment on the engineered organism to prevent its escape-highly control on use of the engineered organism for sustainable biomass production is realized, thereby effectively ensuring the safe and green production of sustainable biomass.

Unless otherwise specified, the experimental methods used in the following examples are conventional methods, which are performed in accordance with the techniques or conditions described in the literature of this field or in accordance with the product specifications. Unless otherwise specified, all materials, reagents and the like used in the following examples are commercially available.

Unless otherwise specified, all quantitative tests in the following examples are conducted with three replicates, and the results are expressed as the mean value.

### Example 1

This Example tested the efficacy of a biological containment method at a transcription-level based on an inducible promoter pGAL1 (Accession No. NM_001178368 in NCBI Database).

### 1.1 Preparation of Promoter Sequence

Inducible promoter sequences corresponding to different first essential genes were prepared via multiple PCR reactions, and fused with a gene sequence of *KanMX* (GenBank: LT726876.1, range: 3800 to 4609, 810 bp in total), which can be used for screening yeast with successful genomic integration in subsequent experiments.
i. Plasmids pXF259 and pXF294 were used as templates to synthesize gene sequences of *KanMX* and promoter pGAL1 through PCR, respectively. During the PCR reaction, homology arms corresponding to the gene for recombination were introduced at 5' end of *KanMX* and 3' end of promoter pGAL1 using primers containing the homology arm sequences for gene recombination. The primer sequences are shown in Table 1, in which lowercase letters indicate homology arm sequences, and uppercase letters indicate sequences binding to the template.

**Table 1**

| DNA sequence | Template | Primer | |
|---|---|---|---|
| *pGAl1* | pXF259 | Forward primer | gaatgctggtcgctatactgGGATGGACGCAAAGAAGTTTAA TAATCATATTAC (SEQ ID No: 1) |
| | | Reverse primer | GTTTTTTCTCCTTGACGTTAAAGTATAGAGG (SEQ ID No: 2) |
| *KanMX* | pXF294 | Forward primer | GACATGGAGGCCCAGAATACCCTC (SEQ ID No: 3) |
| | | Reverse primer | AAACTTCTTTGCGTCCATCCcagtatagcgaccagcattcacata cgattg (SEQ ID No: 4) |

ii. The PCR products obtained in step i were purified and recovered using the Qiagen PCR Product Purification Kit.
iii. OE-PCR primers were designed based on different first essential genes, including *RPC11* (Accession No. NM_001018632 in NCBI Database), *SKP1* (Accession No. NM_001021365 in NCBI Database), and *RPS3* (Accession No. NM_001023783 in NCBI Database). The primer sequences are shown in Tables 2, 3, and 4.

**Table 2**

| Target DNA sequence | Template | Primer | |
|---|---|---|---|
| *KanMX-pGA L-1* | *pGal1-1, KanMX-1* | Forward primer | TTTTGTGTTGCTCATGTCATAGCATTCAACTACA GATGAAgacatggaggcccagaataccctc (SEQ ID No: 5) |
| | | Reverse primer | tcagtagcatattgttacacgaaggacaaaacgaaagcattataGTTTTTT CTCCTTGACGTTAAAGTATAGAGG (SEQ ID No: 6) |

**Table 3**

| Target DNA sequence | Template | Primer | |
|---|---|---|---|
| *KanMX-pG AL-2* | *pGal1-2, KanMX-2* | Forward primer | gacattaccctgcagcgatttaacgggcgtcttggagaaaagaagagaaaG ACATGGAGGCCCAGAATACCCTC (SEQ ID No: 7) |
| | | Reverse primer | gtgaatcgttcaccctcaccactcactaggacaacattagaagtcaccattata GTTTTTTCTCCTTGACGTTAAAGTATAGAGG (SEQ ID No: 8) |

**Table 4**

| Target DNA sequence | Template | Primer | |
|---|---|---|---|
| *KanMX-pG AL-3* | *pGal1-3,K anMX-3* | Forward primer | gtaaattctcttgtttttttttcttgatttcacttcttttcatgttcctttggaataatcG ACATGGAGGCCCAGAATACCCTC (SEQ ID No: 9) |
| | | Reverse primer | ttcagcgtagaagacaccgtcagcgactagctttcttttcttagagattaaagcg accattataGTTTTTTCTCCTTGACGTTAAAGTATAG AGG (SEQ ID No: 10) |

iv. With the primers in step iii, the purified gene sequences of *KanMX* and pGAL promoter from step ii were fused via OE-PCR to obtain fusion fragments KanMX-pGAL1, KanMX-pGAL2, and KanMX-pGAL3, corresponding to different first essential genes. The OE-PCR reaction system and procedures are shown in Tables 5 and 6.

**Table 5**

| Component | Volume |
|---|---|
| ddH₂O | 19 µL |
| 10µM DNA template | 1 µL |
| 10µM primer | 2.5 µL |
| CloneAmp HiFi PCR Premix 2X | 25 µL |

**Table 6**

| **Step** | **Temperature** | **Time** | **Cycle** |
|---|---|---|---|
| 1 | 98 °C | 30 s | 1 |
| 2 | 98 °C | 10 s | 35 |
| 3 | 56 °C | 15 s | |
| 4 | 72 °C | 5 s/kb | |
| 5 | 72 °C | 10 min | 1 |

v. The OE-PCR products obtained in step iv were purified and recovered using the Qiagen PCR Product Purification Kit.

### 1.2 Yeast Transformation

Based on the principle of yeast homologous recombination, the purified fusion fragments KanMX-pGAL1, KanMX-pGAL2, and KanMX-pGAL3 obtained in step v of section 1.1 were separately integrated into the genome of wild-type (wt) yeast. The specific method is as follows.
i. A single colony of wt yeast (deposited in the China Center for Type Culture Collection with an Accession Number of CCTCC NO: M2014434) was inoculated into 4 mL of yeast enrichment medium YPD, and cultured in a shaker at 30 °C and 200 rpm for 24 hours.
ii. 1 mL of the obtained yeast culture in step i was taken to determine the yeast density with a spectrophotometer, where the OD₆₀₀ value was recorded.
iii. According to the OD₆₀₀ value measured in step ii, an appropriate amount of yeast culture was added to a flask containing 20 mL of YPD to adjust the OD₆₀₀ of the yeast culture to 0.1.
iv. The yeast culture from step iii was cultured in a shaker at 30 °C and 200 rpm until the OD₆₀₀ reached 0.6-1.
v. The yeast culture from step iv was centrifuged at 2000 rpm/min for 5 min, to discard the supernatant, and collect the yeast cells.
vi. To the centrifuge tube containing the yeast cells was added sterile ddH₂O (10 mL) to resuspend the cells, followed by centrifugation at 2000 rpm/min for 5 min, to discard the supernatant, and collect the yeast cells.
vii. To the centrifuge tube containing the yeast cells was added LiOAc (0.1 M) to resuspend the cells, followed by centrifugation at 2000 rpm/min for 5 min, to discard the supernatant, and collect the yeast cells.
viii. To the centrifuge tube containing the yeast cells was added LiOAc (0.1 M) to resuspend the cells, yielding competent cells.
ix. The purified fusion fragments KanMX-pGAL1, KanMX-pGAL2, and KanMX-pGAL3 obtained in step v of section 1.1 were individually subjected to the preparation of transformation solutions, which were contained in transformation tubes separately. The transformation solution system is shown in Table 7.

**Table 7**

| Target DNA | X µL (about 200 ng) |
|---|---|
| *ddH₂O* | 22-X µL |
| ssDNA | 50 µL |
| 1 M LioAc | 41 µL |
| 50% PEG3350 | 312 µL |

x. To each transformation tube was added the competent cells (100 µL) prepared in step viii.
xi. The mixture was inverted to mix well and incubated at 30 °C for 30 min.
xii. To the mixture was added DMSO (50 µL), inverting rapidly to mix well.
xiii. The mixture was incubated at 42 °C for 15 min and then placed on ice for 1 min.
xiv. The mixture was centrifuged at 8000 rpm/min for 5 min, to discard the supernatant, and collect the yeast cells.
xv. To the centrifuge tube containing the yeast cells 400 µL of 5 mM CaCl₂ was added to resuspend the cells, followed by incubation at room temperature for 10 min.
xvi. The yeast culture (100 µL) was evenly spread on a drop-out medium and cultured for 2 to 3 days until single colonies grew out.
xvii. The single colonies obtained in step xvi were selected for sequencing verification.
xviii. With correctness verified, strains yXF239, yXF240, and yXF241 for biological containment were obtained.

### 1.3 Determination of Growth Activity

This Example determined the strains obtained in section 1.2 on growth activity. Those skilled in the art will appreciate that the doubling time of cells can be used to reflect the growth activity of the strain: strains with high growth activity have a shorter doubling time, whereas those with low growth activity have a prolonged doubling time. The doubling time of the strain can be accurately calculated by continuous culture and continuous detection of biomass in the culture system, thereby quantifying the growth activity of the strain.
i. The strains for biological containment obtained in section 1.2 were inoculated into liquid drop-out medium (SC+Gal) and cultured in a shaker at 30 °C and 200 rpm until the OD₆₀₀ reached 2 to 3.
ii. The yeast culture was transferred into a culture well, and liquid drop-out medium was added to adjust the culture to a final OD₆₀₀ of 0.01 and a final volume of 200 µL per well.
iii. The well plate was placed in a Bioscreen continuous incubator for cultivation and data collection. The program settings of the incubator are shown in Table 8.

**Table 8**

| | |
|---|---|
| Culture time | 48 H |
| Culture temperature | 30 °C |
| Shaking amplitude | medium |
| Shaking frequency | 1 shaking/min |
| Shaking time | 15 s |
| Detection wavelength | 600 nm |
| Detection time | 10 s after the shaking |
| Detection frequency | per 20 min |

iv. The doubling time of each strain was calculated based on the collected data, with reference to the method described in relevant literature (Fernandez-Ricaud et al. BMC Bioinformatics (2016) 17:249).

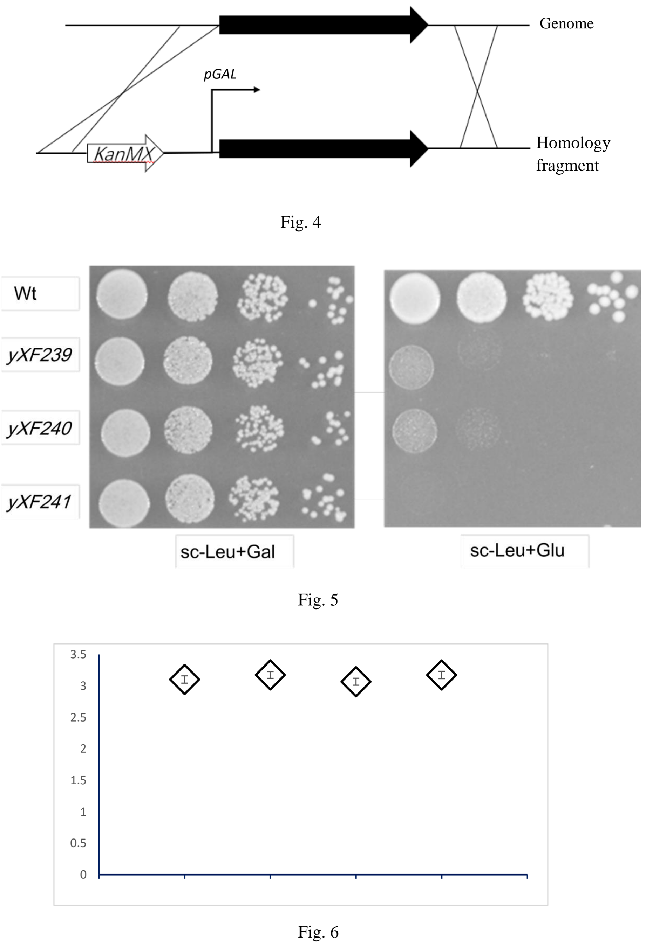

In this Example, the doubling time of yeast with the wt genome was measured to be 3.1 hours, while that of yXF239 was 3.2 hours, yXF240 was 3.1 hours, and yXF241 was 3.2 hours. These results of growth activity determination for the strains for biological containment indicate that replacing the promoter of the essential gene with the inducible promoter pGAL had no significant effect on strain growth (Fig. 6). In other words, after promoter replacement with the inducible promoter, the strains for biological containment in this Example maintained their original viability, demonstrating the feasibility of subsequent biological containment based on the introduction of the inducible promoter.

### 1.4 Verification of Biological Containment Efficacy

This Example performed qualitative and quantitative verification of biological containment efficacy for the strains obtained in section 1.2. The medium supplemented with an inducer SC+Gal (galactose) was defined as a permissive condition, while SC+Glu medium was defined as a non-permissive condition.

### 1.4.1 Qualitative Verification of Biological Containment Efficacy

The strains yXF239, yXF240, and yXF241 were separately spread on SC+Gal and SC+Glu solid media at serial dilutions and cultured at 30 °C for 2 days. The results of qualitative verification of biological containment efficacy are shown in Fig. 5, where strains yXF239, yXF240, and yXF241 grew normally under the permissive condition, whereas their growth was inhibited under the non-permissive condition. These results indicates that the inducible promoter used in this Example achieved the desired biological containment effect.

### 1.4.2 Quantitative Verification of Biological Containment Efficacy

This Example performed the quantitative verification of biological containment efficacy via escape rate assays. Those skilled in the art will understand that there is a correlation between the volume of the microbial culture and the inoculation amount. When cells in the solution are plated under permissive conditions, all cells can grow into colonies on the plate; whereas cells that grow under non-permissive conditions have evaded the biological containment. Therefore, the escape rate can be calculated by using the number of colonies grown on plates inoculated with a large volume of microbial culture under non-permissive conditions as the denominator, and the number of colonies grown on plates inoculated with a quantified volume of microbial culture under permissive conditions as the numerator. For example, a specific escape rate calculation method was as follows: after inoculating 10⁻⁴ mL of microbial culture onto SC solid medium plates and culturing for 2 days, the number of single colonies grown is x; after 8 days of culture, no single colonies grow on SC-Leu-Ura solid medium plates inoculated with 10⁻² mL or 10⁻¹ mL of microbial culture, while single colonies grow on solid medium plates lacking unnatural amino acids inoculated with 1 mL and 10 mL of microbial culture, then taking the plate inoculated with the minimum volume (i.e., 1 mL) of microbial culture that allows colony growth for counting, the number of single colonies of which is recorded as y. The escape rate of this strain is calculated by the formula: Escape Rate = (y × 10⁻⁴) / (x × 1).
i. The strains yXF239, yXF240, and yXF241 were separately inoculated into SC+Gal liquid medium at an initial OD₆₀₀ of 0.1, and cultured in a shaker at 30 °C and 200 rpm until the late logarithmic phase (OD₆₀₀ ≈ 2-3).
ii. The yeast cells were washed three times and then diluted to an OD₆₀₀ of 1.
iii. Yeast cultures (10⁻⁴ mL) of yXF239, yXF240, and yXF241 were separately spread on SC+Gal solid medium plates, and cultured in an incubator at 30 °C for 2 days under the permissive condition.
iv. The number of colonies grown on the plates was counted and recorded as x.
v. Yeast cultures of yXF239, yXF240, and yXF241 were separately spread on SC+Glu solid medium plates at inoculation volumes of 10⁻² mL, 10⁻¹ mL, 1 mL, and 10 mL, and cultured in an incubator at 30 °C for 8 days under the non-permissive condition.
vi. The minimum inoculation volume n that allows colony growth was determined, and the number of colonies grown on the corresponding plates was counted and recorded as y.
vii. The escape rate of each strain was calculated with the formula: Escape Rate = (y × n) / (x × 1). The results are shown in Table 9.

**Table 9**

| **Strain** | yXF239 | yXF240 | yXF241 |
|---|---|---|---|
| **Escape Rate** | 2.79E-04 | 2.17E-06 | 2.41E-07 |

As shown in Table 9, all transcriptionally regulated strains exhibited biological containment efficacy. Among them, yXF241 showed the best containment efficacy, with an escape rate reaching the magnitude of 1E-7, presenting its feasibility for biological containment applications.

This Example 1 of the present disclosure replaced the promoter of the essential gene with an inducible pGAL promoter to construct a switch for controlling the essential gene's expression, and regulated the expression of the target essential gene by controlling the introduction of the promoter inducer Gal, thereby achieving the biological containment. This Example tested three different essential genes, and the results showed that the introduction of the pGAL promoter successfully constructed strains with transcription-level biological containment without significantly affecting strain growth. To better evaluate the efficacy of this method, this Example tested the escape rates of these transcriptionally regulated strains. It was found that replacing the native promoter of the gene *RPS3* with the inducible pGAL promoter achieved the optimal biological containment effect. Accordingly, the inducible pGAL promoter can effectively inhibit the growth of engineered organisms at the transcriptional level, providing an efficient biological containment method.

### Example 2

This Example tested the efficacy of a biological containment method at a transcription-translation co-regulation level based on an inducible promoter pGAL1 and the introduction of termination codons. Specifically, a methoxytyrosyl-tRNA synthetase/leucine tRNA orthogonal pair (LeuOmeRS pair, LeuOmeRS/tRNA_{CUA}, ACS Synth. Biol. 2018, 7, 9, 2256-2269) was used as an unnatural-amino-acid encoding tool. Namely, the test was performed on the basis of the introduction of the inducible promoter by providing a vector pXF231 containing the LeuOmeRS/tRNA_{CUA}, and selecting amino acids F9, P5, H520 and F325 in proteins encoded by yeast essential genes *CDC27* and *CDC4* as the insertion sites for the unnatural amino acid, where the coding sequences of wt CDC27 and CDC4 are set forth in SEQ ID No: 11 and SEQ ID No: 12, respectively.
wt CDC27 coding sequences (SEQ ID No: 11): 
MAVNPELAPFTLSRGIPSFDDQALSTIIQLQDCIQQAIQQLNYSTAEFLAELLYAECSILDKSSVYWSDA
VYLYALSLFLNKSYHTAFQISKEFKEYHLGIAYIFGRCALQLSQGVNEAILTLLSIINVFSSNSSNTRINMVL
NSNLVHIPDLATLNCLLGNLYMKLDHSKEGAFYHSEALAINPYLWESYEAICKMRATVDLKRVFFDIAGK
KSNSHNNNAASSFPSTSLSHFEPRSQPSLYSKTNKNGNNNINNNVNTLFQSSNSPPSTSASSFSSIQHFSRSQ
QQQANTSIRTCQNKNTQTPKNPAINSKTSSALPNNISMNLVSPSSKQPTISSLAKVYNRNKLLTTPPSKLLN
NDRNHQNNNNNNNNNNNNNNNNNNNNNNNNNIINKTTFKTPRNLYSSTGRLTTSKKNPRSLIISNSILTSD
YSITLPEIMYNFALILRSSSQYNSFKAIRLFESQIPSHIKDTMPWCLVQLGKLHFEIINYDMSLKYFNRLKDL
QPARVKDMEIFSTLLWHLHDKVKSSNLANGLMDTMPNKPETWCCIGNLLSLQKDHDAAIKAFEKATQLD
PNFAYAYTLQGHEHSSNDSSDSAKTCYRKALACDPQHYNAYYGLGTSAMKLGQYEEALLYFEKARSINP
VNVVLICCCGGSLEKLGYKEKALQYYELACHLQPTSSLSKYKMGQLLYSMTRYNVALQTFEELVKLVPD
DATAHYLLGQTYRIVGRKKDAIKELTVAMNLDPKGNQVIIDELQKCHMQE.
wt CDC4 coding sequences (SEQ ID No: 12):

MGSFPLAEFPLRDIPVPYSYRVSGGIASSGSVTALVTAAGTHRNSSTAKTVETEDGEEDIDEYQRKRA
AGSGESTPERSDFKRVKHDNHKTLHPVNLQNTGAASVDNDGLHNLTDISNDAEKLLMSVDDGSAAPSTL
SVNMGVASHNVAAPTTVNAATITGSDVSNNVNSATINNPMEEGALPLSPTASSPGTTTPLAKTTKTINNNN
NIADLIESKDSIISPEYLSDEIFTAINNNLPHAYFKNLLFRLVANMDRSELSDLGTLIKDNLKRDLITSLPFEIS
LKIFNYLQFEDIINSLGVSQNWNKIIRKSTSLWKKLLISENFVSPKGFNSLNLKLSQKYPKLSQQDRLRLSF
LENIFILKNWYNPKFVPQRTTLRGHMTSVITCLQFEDNYVITGADDKMIRVYDSINKKFLLQLSGHDGGV
WALKYAHGGILVSGSTDRTVRVWDIKKGCCTHVFKGHNSTVRCLDIVEYKNIKYIVTGSRDNTLHVWKL
PKESSVPDHGEEHGYPLVFHTPEENPYFVGVLRGHMASVRTVSGHGNIVVSGSYDNTLIVWDVAQMKCL
YILSGHTDRIYSTIYDHERKRCISASMDTTIRIWDLENIWNNGECSYATNSASPCAKILGAMYTLQGHTALV
GLLRLSDKFLVSAAADGSIRGWDANDYSRKFSYHHTNLSAITTFYVSDNILVSGSENQFNIYNLRSGKLVH
ANILKDADQIWSVNFKGKTLVAAVEKDGQSFLEILDFSKASKINYVSNPVNSSSSSSSLESISTSLGLTRTTII
P.

### 2.1 Generation of Transcription-Translation Co-Regulated Strains

i. With yXF241 as a template, a purified fusion fragment KanMX-pGAL was prepared (refer to section 1.1 in Example 1 for specific steps and procedures). The PCR primer sequences are shown in Table 10.

**Table 10**

| **Target DNA** | **Template** | | **Sequence** |
|---|---|---|---|
| ***KanMX-pGAL1*** | *yXF241* | Forward primer | GTAAATTCTCTTGTTTTTTTTTCTTGATTTCACTTCTT TTCATGTTCCTTTGGAATAATCgacatggaggcccagaataccct c (SEQ ID No: 9) |
| | | Reverse primer | TTCAGCGTAGAAGACACCGTCAGCGACTAGCTTTCT TTTCTTAGAGATTAAAGCGACCATTATAGTTTTTTCT CCTTGACGTTAAAGTATAGAGG (SEQ ID No: 10) |

| | | | |
|---|---|---|---|
| Note: The underlined regions are homologous to gene *RPS3.* | | | |

ii. Through yeast homologous recombination, the purified fusion fragment KanMX-pGAL was separately integrated into the genome of yeast yXF222, yXF098, yXF218 and yXF097 (refer to section 1.2 of Example 1 for details). In comparison with the wt yeast, yXF222 was modified by replacing the 9th amino acid (F) from the N-terminus encoded by *CDC4* with a first termination codon (i.e., a codon for the unnatural amino acid), yielding a gene sequence *CDC4*(F9amb) carrying a "TAG" codon. Similarly, in yXF098, the 325th amino acid (F) from the N-terminus encoded by wt *CDC4* was replaced with the first termination codon (i.e., a codon for the unnatural amino acid), generating a gene sequence *CDC4*(F325amb) carrying a "TAG" codon. Analogously, yXF218 was modified to obtain a gene sequence *CDC27*(P5amb) carrying a "TAG" codon, and yXF097 was modified to obtain a gene sequence *CDC27*(H520amb) carrying a "TAG" codon.
iii. The strains were subjected to sequencing. With correctness verified, transcription-translation co-regulated strains yXF251, yXF252, yXF253 and yXF254 were obtained (see Table 16 for the correspondence).

### 2.2 Determination of Growth Activity

This Example determined the strains obtained in section 2.2 on growth activity. Refer to section 1.3 in Example 1 for the method for determining growth activity.

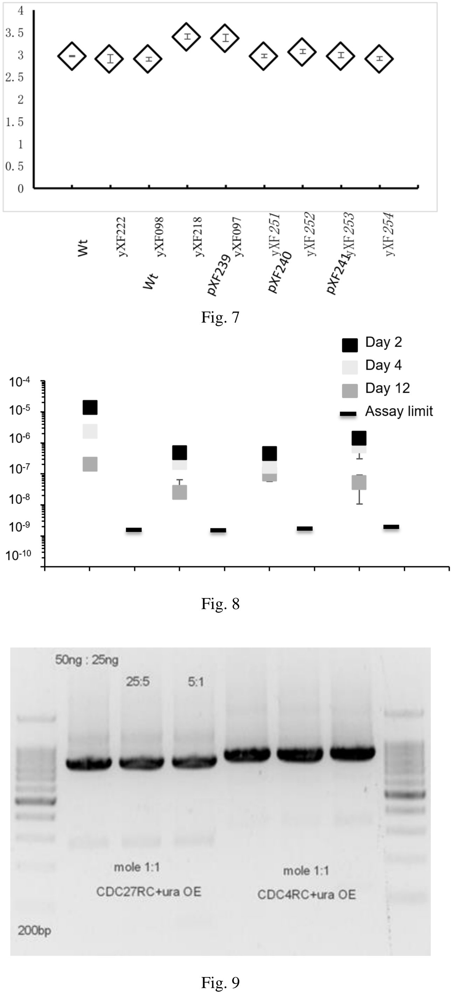

Fig. 7 shows results of growth activity determination for the transcription-translation co-regulated strains. As shown in Fig.7, there was no significant difference between the doubling time of yeast with the wt genome and that of yXF251, yXF252, yXF253 and yXF254. These results demonstrate that the modifications at both transcriptional and translational levels in this example did not affect the normal growth activity of the strains, thus verifying the feasibility of biological containment strategy for the transcription-translation co-regulation.

### 2.3 Verification of Biological Containment Efficacy

This Example performed qualitative and quantitative verification of biological containment efficacy for the strains obtained in section 2.2. The medium supplemented with an inducer, i.e. SC-leu+OmeY+Gal, was defined as a permissive condition, while SC+leu medium was defined as a non-permissive condition.

### 2.3.1 Qualitative Verification of Biological Containment Efficacy

The strains yXF251, yXF252, yXF253 and yXF254 were separately spread on SC-leu+OmeY+Gal and SC+leu solid media at serial dilutions. Refer to section 1.4.1 of Example 1 for the specific procedure of qualitative verification of biological containment efficacy.

The results of qualitative verification of biological containment efficacy are shown in Fig. 8, where no single colonies of strains yXF251, yXF252, yXF253 and yXF254 grew under the non-permissive condition, indicating an excellent biological containment effect.

### 2.3.2 Quantitative Verification of Biological Containment Efficacy

Referring to the steps in section 1.4.2 of Example 1, strains yXF251, yXF252, yXF253 and yXF254 were subjected to quantitative verification of biological containment efficacy, where n=100 mL (viable cell count ≈ 10⁹).

**Table 11**

| **Strain** | *yXF251* | *yXF252* | *yXF253* | *yXF254* |
|---|---|---|---|---|
| **Colony count** | 0 | 0 | 0 | 0 |

The quantitative verification results of biological containment efficacy are shown in Table 11. In the case of using a biological containment method of transcription-translation co-regulation, no colonies grew under the non-permissive condition, even with a viable cell count for plating reaching 10⁹ (the assay limit).

This example, based on the introduction of an inducible promoter pGAL1 and termination codons, co-regulates two different essential genes at both the transcriptional and translational levels, constructing a biological containment strategy of transcription-translation co-regulation. This strategy did not affect the normal growth of the strains under the permissive condition (medium supplemented with both OmeY and galactose), and could inhibit the growth of the target yeast strains under the non-permissive condition (medium lacking both OmeY and galactose, and supplemented with glucose). No escaped colonies were detected even at the assay limit of the existing test method, achieving an excellent biological containment effect.

### Example 3

In the related art, there are challenges for identifying essential genes and their specific substitution sites, which require sophisticated and elaborate design or extensive testing. The Example of the present disclosure constructed an N-terminal library of the target protein, and such a method enables rapid and efficient identification of essential genes and their specific substitution sites with minimal impact on strain growth. The specific operations are as follows.

### 3.1 Preparation of Transcriptionally Regulated Strain

The transcriptionally regulated strain yXF241 was prepared, which was verified to exhibit the optimal escape-prevention efficacy among all transcriptionally regulated strains (see Example 1 for details).

### 3.2 Preparation of Terminator Sequence

In this example, 17 codon positions from N4 to D20 and from F4 to Y20 within the N-terminal regions of the proteins encoded by the yeast essential gene *CDC27* and *CDC4,* respectively, were selected as insertion sites for an unnatural amino acid (TAG). Terminator sequences located at the corresponding coding positions were constructed.
i. With a plasmid pXF414 as a template, respective 17 gene sequences of *CDC27* and *CDC4* were synthesized by PCR. In these sequences, the nucleotide sequence corresponding to each of the 17 codons (N4 to D20 for CDC27 and F4 to Y20 for CDC4) in the N-terminal region of the encoded protein were replaced one by one with the terminator TAG. The PCR reaction system and procedures are shown in Tables 13 and 14.

**Table 13**

| Component | Volume |
|---|---|
| ddH₂O | 9.5 µL |
| 20 ng DNA template | 1 µL |
| 10µM forward and reverse primers | 2 µL |
| Q5^{®} Hot Start High-Fidelity 2X Master Mix | 12.5 µL |

**Table 14**

| Step | Temperature | Time | Cycle |
|---|---|---|---|
| 1 | 98 °C | 30 s | 1 |
| 2 | 98 °C | 10 s | 35 |
| 3 | 62 °C | 15 s | |
| 4 | 72 °C | 1 min/kb | |
| 5 | 72 °C | 2 min | 1 |

ii. With a strain inserted with *URA3* at upstream of genes *CDC4* and *CDC27* as a template, a gene sequence of *Ura3* (GenBank: MG833229.1, Range: 171 to 1341, 1171 bp in total) was obtained, which served as a selection marker for genomic integration. The primer sequences used in steps i and ii are shown in Table 15.

**Table 15**

| **CDC4** | **Forward primer** | **5'-CTGTGTGCTTAAAATATAGTGTCTTTTTTGGG-3' (SEQ ID No: 13)** |
|---|---|---|
| | Reverse primer | 5'-CTTCTGTCTCAACTGTCTTAGCC-3' (SEQ ID No: 14) |
| CDC4-URA3 | Forward primer | 5'-CCACTGAGAGCTGTTCGTAGATAAATC-3' (SEQ ID No: 15) |
| | Reverse primer | 5'-CCCAAAAAAGACACTATATTTTAAGCACACAG-3' (SEQ ID No: 16) |
| **CDC27** | Forward primer | 5'-GGATCAAAGTGCAAGCAAAGGTG-3' (SEQ ID No: 17) |
| | Reverse primer | 5'-CTTGATTTATCGAGAATGGAGCATTCAGC-3' (SEQ ID No: 18) |
| CDC27-URA3 | Forward primer | 5'-CTCGCCAAAAGCCATCTTCGTAC-3' (SEQ ID No: 19) |
| | Reverse primer | 5'-CACCTTTGCTTGCACTTTGATCC-3' (SEQ ID No: 20) |

iii. The PCR products of steps i and ii were purified and recovered using the Macherey-Nagel PCR Product Purification Kit.
iv. The gene sequences of *CDC27* or *CDC4* each were fused with the purified gene sequence of *Ura3* obtained in step iii via OE-PCR, obtaining *CDC27-Ura3* or *CDC4-Ura3* sequences.
v. The PCR products of step iv were purified and recovered using the Macherey-Nagel PCR Product Purification Kit. The gel electrophoresis image of the PCR products is shown in Fig. 9.

### 3.3 Construction of N-Terminal Library of the Target Protein

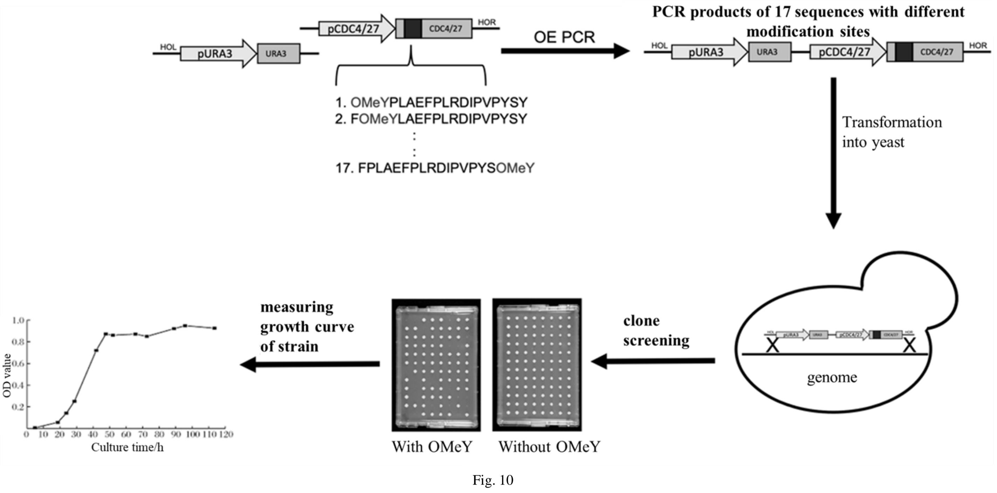

In this Example, through yeast homologous recombination, the respective 17 gene sequences of *CDC27-Ura3* and *CDC4-Ura3* were separately integrated into the genome of yeast BY4741-pGAL-RPS3 (yXF241). Clones with successful genomic integration were selected using URA3. Refer to Section 1.2 of Example 1 for the specific method. A schematic diagram of genomic integration is shown in Fig. 10.

### 3.4 Determination of Growth Activity

i. The strains obtained in section 3.3 were plated onto a non-permissive condition (absence of OmeY), where none of the clones with successful replacement of amino acid coding sequences by the terminator TAG could grow. In contrast, these clones grew normally under a permissive condition (supplemented with OmeY). These results indicate that the biological containment strategy in this example is effective.
ii. A total of 124 *CDC4*-modified strains and 146 *CDC27*-modified strains were selected from the N-terminal mutant library of the target protein for sequencing.
iii. The strains obtained in section 3.3 were adjusted to an OD₆₀₀ of 0.01 and inoculated into SC-Ura-Leu+OmeY liquid medium. The cultures were incubated at 30 °C in a Bioscreen growth curve analyzer, with OD₆₀₀ measurements taken every 20 minutes until the strains reached the stationary phase, for a total measurement duration of 48 hours. Growth curves of all selected strains were plotted. The doubling time of the strains was calculated with reference to the literature (PRECOG: A tool for automated extraction and visualization of fitness components in microbial growth phenomics, BMC Bioinformatics. 2016. 17:249).

### Results

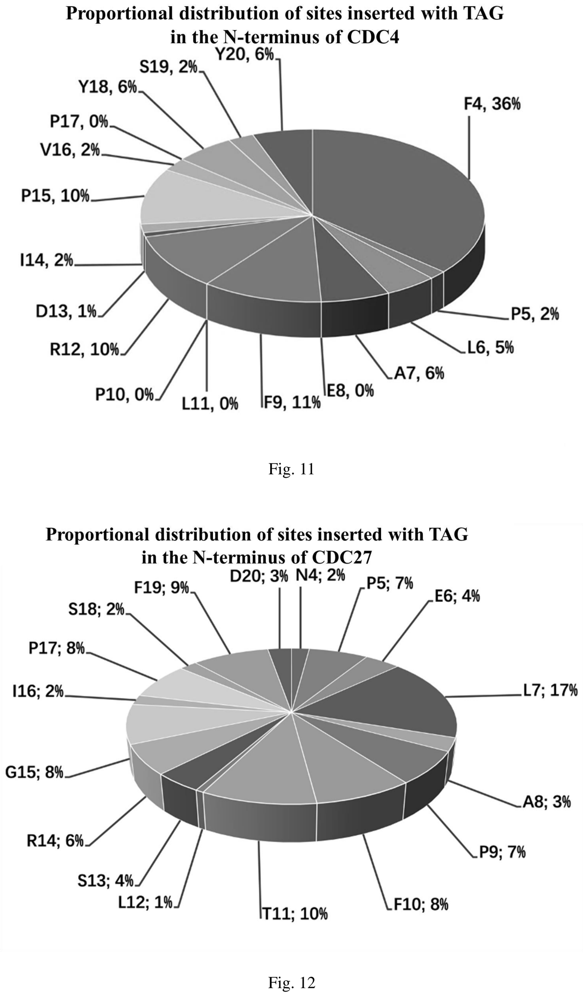

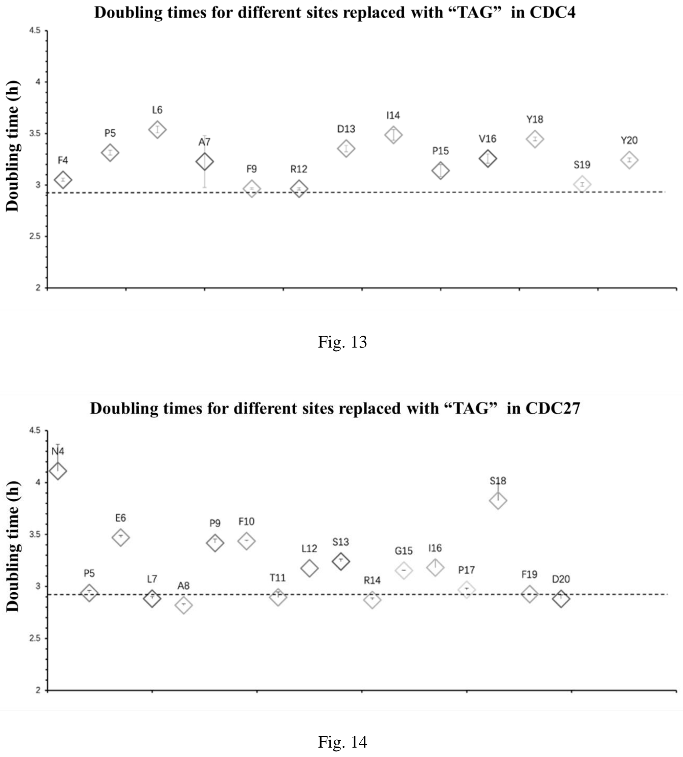

Sanger sequencing showed that the CDC4 and CDC27 mutant libraries after transformation substantially covered the intended diversity, as illustrated in Figs. 11 and 12. These results demonstrate that the mutant library construction method proposed in the Example significantly improves the screening basis and efficiency for biological containment systems. In addition, the doubling time of the initial strain was calculated to be 2.9 hours, while the doubling times of the other strains are shown in Figs. 13 and 14. The growth curve measurements indicate that, on the basis of ensuring the efficacy of the biological containment strategy of transcription-translation co-regulation, the method proposed in the example enables the acquisition of escape-resistant strains with low escape rates and robust growth performance by further testing and screening sites within N-terminal of a protein suitable for unnatural amino acid insertion. Moreover, from data analysis, the growth of these strains was almost identical to that of the original strain, indicating that the viability of the strains identified from the mutant library constructed in this example was not compromised by the stress imposed by exogenous promoter elements and unnatural amino acids, presenting well applicability for bioproduction.

In the screening on the N-terminal library of CDC4, three sites-F9, R12 and S19-maintained doubling times similar to that of the original strain after insertion of the unnatural amino acid. In the screening on the N-terminal library of CDC27, 8 out of the 17 sites, i.e., P5, L7, A8, T11, R14, P17, F9 and D20 in the library exhibited growth rates comparable to that of the original strain after unnatural amino acid insertion. These results indicate that by constructing an N-terminal library of a target protein, the method in the Example can rapidly identify essential genes and their specific substitution sites through screening on N-terminus library of a protein, without relying on complex prior design. This method is of great significance for the biosafety of engineered organisms.

The information of the strains used in the Examples of the present disclosure is shown in Table 16.

**Table 16**

| **Strain** | **Brief information** | **Source** |
|---|---|---|
| Wt | *MATa his3Δ1 leu2Δ0 met15Δ0 ura3Δ0 [pXF231]* | The present research |
| Wt-A | *MATa his3Δ1 leu2Δ0 met15Δ0 ura3Δ0 YBL084C:: URA3 [pXF231]* | The present research |
| WT-B | *MATa his3Δ1 leu2Δ0 met15Δ0 ura3Δ0 YBL084C:: URA3 YFL009W::HIS3 [pXF231]* | The present research |
| yXF083 | *MATa his3Δ1 leu2Δ0 ura3Δ0 LYS2 MET5 can1Δ::BleoR YFL009W-ts::URA3* | The present research |
| yXF082 | *MATa his3Δ1 leu2Δ0 met15Δ0 ura3Δ0 YBL084C-ts::KanMX (G1838A;A1839C)* | The present research |
| yXF097 | *MATa his3Δ1 leu2Δ0 met15Δ0 ura3Δ0 YBL084C:: URA3(H520amb) [pXF231]* | The present research |
| yXF098 | *MATa his3Δ1 leu2Δ0 met15Δ0 ura3Δ0 YFL009W::URA3 (F325amb) [pXF231]* | The present research |
| yXF099 | *MATa his3Δ1 leu2Δ0 met15Δ0 ura3Δ0 YBL084C:: URA3(H520amb) YFL009W:: HIS3 (F325amb ) [pXF231]* | The present research |
| yXF218 | *MATa his3Δ1 leu2Δ0 met15Δ0 ura3Δ0 YBL084C:: URA3(P5amb ) [pXF231]* | The present research |
| yXF222 | *MATa his3Δ1 leu2Δ0 met15Δ0 ura3Δ0 YFL009W::URA3 (F9amb) [pXF231]* | The present research |
| yXF239 | *MATa his341 leu2Δ0 met15Δ0 ura3Δ0 KanMX::pGAL::RPC11 [pXF231]* | The present research |
| yXF240 | *MATa his3Δ1 leu2Δ0 met15Δ0 ura3Δ0 KanMX::pGAL:: SKP1 [pXF231]* | The present research |
| yXF241 | *MATa his3Δ1 leu2Δ0 met15Δ0 ura3Δ0 KanMX::pGAL:: RPS3 [pXF231]* | The present research |
| yXF251 | *MATa his3Δ1 leu2Δ0 met15Δ0 ura3Δ0 KanMX::pGAL::RPS3 YFL009W::URA3 (F9amb ) [pXF231]* | The present research |
| yXF252 | *MATa his3Δ1 leu2Δ0 met15Δ0 ura3Δ0 KanMX::pGAL::RPS3 YFL009W::URA3 (F325amb ) [pXF231]* | The present research |
| yXF253 | *MATa his3Δ1 leu2Δ0 met15Δ0 ura3Δ0 KanMX::pGAL::RPS3 YBL084C:: URA3(P5amb ) [pXF231]* | The present research |
| yXF254 | *MATa his3Δ1 leu2Δ0 met15Δ0 ura3Δ0 KanMX::pGAL::RPS3 YBL084C:: URA3(H520amb ) [pXF231]* | The present research |

The information of the plasmids used in the Examples of the present disclosure is shown in Table 17.

**Table 17**

| **Plasmid** | **Brief information** | **Source** |
|---|---|---|
| pXF231 | LeuOmeRS pair | ACS Synth. Biol. 2018, 7, 9, 2256-2269 |
| pXF412 | pRS416-CDC27 | The present research |
| pXF413 | pRS413-*CDC4* | The present research |
| pXF414 | *pRS416-CDC27(H520amb)* | The present research |
| pXF415 | *pRS413-CDC4(F325amb)* | The present research |
| pXF294 | CRISPEY with KanMX | The present research |
| pXF259 | CRISPEY with pGAL1/10 | The present research |

The information of the apparatus used in the Examples of the present disclosure is shown in Table 18.

**Table 18**

| **Apparatus** | **Manufacturer** |
|---|---|
| **SW-CJ-2FD Clean Bench** | SUZHOU ANTAI AIRTECH CO., LTD |
| Pipettor | Eppendorf |
| **BSA223S-CW Analytical Balance (0.001 g precision)** | Sartorius |
| SPX-350L Biochemical Incubator | Zhongyiguoke (Beijing) Technologies Co., Ltd |
| **ZQZY-CS8 Triple-layer Stackable Shaking Incubator** | Shanghai Zhichu Instrument Co., Ltd. |
| **Gel DocTM XR+ Gel Imaging System** | Biorad |
| PCR Amplifier | Trobot |
| **Nanodrop2000 Microvolume UV-Vis Spectrophotometer** | Thermo Fisher |
| 5418 Benchtop Centrifuge | Eppendorf |
| Bioscreen Growth Curve Analyzer | Bioscreen (Finland) |
| **L535R-1 Low-Speed Refrigerated Centrifuge** | Hunan Xiangyi Laboratory Instrument Development Co., Ltd |
| **DK-S26 Electric Thermostatic Water Bath** | Shanghai Jinghong Experimental Equipment Co., Ltd. |

The preparation methods of the reagents used in the Examples of the present disclosure are as follows.
50xOmeY: Weigh 0.5 g of OmeY and dissolve in 50 mL of ultrapure water; sterilize by filtration, where OmeY cannot be sterilized by autoclaving.
1000×Carb (100 mg/mL): Weigh 1 g of Carb and dissolve in 10 mL of ultrapure water; sterilize by filtration.
2 ×Agar (4%): Weigh 10 g of Agar and dissolve with water to a final volume of 250 mL.
40% Glucose: Weigh 200 g of glucose and dissolve with water to a final volume of 500 mL.
100 ×Ura: Weigh 2.24 g of Ura and dissolve with water to a final volume of 1 L.
100 ×His: Weigh 21 g of His and dissolve with water to a final volume of 1 L.
50 ×Leu: Weigh 13 g of Leu and dissolve with water to a final volume of 1 L.
2×SC-3: Dissolve 3.4 g of Yeast Nitrogen Base w/o Amino Acids and Ammonium Sulfate, 1.3 g of Amino Acid Drop-out Mix (Clontech Aamix) and 10 g of ammonium sulfate with water to a final volume of 1 L.
SC-Ura liquid medium: Combine 250 mL of 2×SC-3, 25 g of 40% glucose, 5 mL of 100×His and 10 mL of 50 ×Leu; add water to a final volume of 500 mL.
SC-Ura solid medium: Combine 250 mL of 2×SC-3, 25 g of 40% glucose, 5 mL of 100×His and 10 mL of 50 ×Leu; add 2 ×Agar (4%) to a final volume of 500 mL.
SC-His liquid medium: Combine 250 mL of 2×SC-3, 25 g of 40% glucose, 5 mL of 100×Ura and 10 mL of 50×Leu; add water to a final volume of 500 mL.
SC-His solid medium: Combine 250 mL of 2×SC-3, 25 g of 40% glucose, 5 mL of 100×Ura and 10 mL of 50×Leu; add 2×Agar (4%) to a final volume of 500 mL.
SC-Leu-Ura liquid medium: Combine 250 mL of 2×SC-3, 25 g of 40% glucose and 5 mL of 100×His; add water to a final volume of 500 mL.
SC-Leu-Ura solid medium: Combine 250 mL of 2×SC-3, 25 g of 40% glucose and 5 mL of 100×His; add 2 ×Agar (4%) to a final volume of 500 mL.
SC-Leu-Ura+OmeY liquid medium: Combine 250 mL of 2×SC-3, 25 g of 40% glucose, 5 mL of 100×His and 10 mL of 50 ×OmeY; add water to a final volume of 500 mL.
SC-Leu-Ura+OmeY solid medium: Combine 250 mL of 2×SC-3, 25 g of 40% glucose, 5 mL of 100×His and 10 mL of 50 ×OmeY; add 2×Agar (4%) to a final volume of 500 mL.
SC-3+OmeY liquid medium: Combine 250 mL of 2×SC-3, 25 g of 40% glucose and 10 mL of 50 ×OmeY; add water to a final volume of 500 mL.
SC-3 solid medium: Combine 250 mL of 2×SC-3 and 25 g of 40% glucose; add 2×Agar (4%) to a final volume of 500 mL.
LB liquid medium: Weigh 10 g of LB Broth and add water to a final volume of 400 mL.
LB+Carb solid medium: Combine 10 g of LB Broth, 6 g of agar and 0.4 mL of 1000×Carb; add water to a final volume of 400 mL.
LB+Carb liquid medium: Combine 10 g of LB Broth and 0.4 mL of 1000 ×Carb; add water to a final volume of 400 mL.

All the above reagents were sterilized at 121 °C for 20 minutes.

Reference throughout this specification to terms "an embodiment", "some embodiments", "an example", "a specific example" or "some examples" means that a particular feature, structure, material, or characteristic described in connection with the embodiment or example is included in at least one embodiment or example of the present disclosure. Thus, the appearances of the above terms, in various places throughout this specification are not necessarily referring to the same embodiment or example of the present disclosure. Furthermore, the particular features, structures, materials, or characteristics may be combined in any suitable manner in one or more embodiments or examples. Besides, any different embodiments and examples and any different features of embodiments and examples may be combined by those skilled in the art without contradiction.

Although explanatory embodiments have been shown and described, it would be appreciated that the above embodiments are exemplary and cannot be construed to limit the present disclosure, and that changes, alternatives, and modifications within the scope of the present disclosure can be made to the embodiments by those skilled in the art.

## Claims

1. An engineered biological containment system, comprising:
a first nucleic acid, comprising at least a partial sequence of a first essential gene;
a second nucleic acid, comprising at least a partial sequence of a second essential gene;
a third nucleic acid activating the transcription of the first nucleic acid; and
a fourth nucleic acid, encoding an unnatural-amino-acid encoding tool;
wherein the second nucleic acid comprises one or more nucleotide sequences encoding first termination codons, and optionally comprises a nucleotide sequence encoding a second termination codon located at the end of a coding region of the second essential gene,
optionally, the first essential gene is the same as or different from the second essential gene.

2. The engineered biological containment system of claim 1, wherein the third nucleic acid is an inducible promoter, preferably an inducible promoter pGAL and/or CaMV35S.

3. The engineered biological containment system of claim 1, wherein the first termination codon encodes an unnatural amino acid.

4. The engineered biological containment system of claim 1, wherein the nucleotide sequence encoding the first termination codon replaces a nucleotide sequence encoding one or more amino acids among the first 20 amino acids at the N-terminus of a protein encoded by the second essential gene,
preferably, the nucleotide sequence encoding the first termination codon replaces a nucleotide sequence encoding one or more amino acids among the 4th to the 20th amino acids at the N-terminus of the protein encoded by the second essential gene.

5. The engineered biological containment system of claim 4, wherein the second essential gene comprises a gene with a medium-to-low expression level,
optionally, the second essential gene comprises at least one of *CDC27* and *CDC4.*

6. The engineered biological containment system of claim 5, wherein
the nucleotide sequence encoding the first termination codon replaces a nucleotide sequence encoding any one or a combination of the following amino acids of *CDC4:* F4, P5, L6, A7, E8, F9, P10, L11, R12, D13, I14, P15, V16, P17, Y18, S19 and Y20; and/or
the nucleotide sequence encoding the first termination codon replaces a nucleotide sequence encoding any one or a combination of the following amino acids of *CDC27:* N4, P5, E6, L7, A8, P9, F10, T11, L12, S13, R14, G15, I16, P17, S18, F19 and D20,
preferably, the nucleotide sequence encoding the first termination codon replaces a nucleotide sequence encoding any one or a combination of the following amino acids of *CDC4:* F9, R12 and S19;
preferably, the nucleotide sequence encoding the first termination codon replaces a nucleotide sequence encoding any one or a combination of the following amino acids of *CDC27:* P5, L7, A8, T11, R14, P17, F9 and D20.

7. The engineered biological containment system of claim 1, wherein the second essential gene comprises at least one of *CDC27* and *CDC4,* wherein
the nucleotide sequence encoding the first termination codon replaces a nucleotide sequence encoding the 520th amino acid of *CDC27;* and/or
the nucleotide sequence encoding the first termination codon replaces a nucleotide sequence encoding the 325th amino acid of *CDC4.*

8. The engineered biological containment system of any one of claims 1 to 7, wherein the unnatural-amino-acid encoding tool comprises a methoxytyrosyl-tRNA synthetase/leucine tRNA orthogonal pair (LeuOmeRS/tRNA_{CUA}), and
the unnatural amino acid encoded by the first termination codon comprises *O*-methyl-L-tyrosine (OmeY).

9. The engineered biological containment system of claim 1, wherein the first essential gene is selected from the group consisting of *RPC11, SKP1* and *RPS3.*

10. The engineered biological containment system of any one of claims 1 to 9, further comprising a vector selected from the group consisting of a eukaryotic cell expression vector and a prokaryotic cell expression vector,
optionally, the eukaryotic cell expression vector comprising: pXF412, pXF413, pXF414, pXF415, pXF294, pXF259, pDR196, pHISi, pESP-3, pESP-2, pESP-1, pHiSi-1, pGAG424, p53his, pRS426gal, pRS41H, pRS413, pRS416, pGBT9 and pAUR123,
optionally, the prokaryotic cell expression vector comprising: pET28a-TagRFP-N, pTrc-CKS, pET-DsbA, pET-Trx, pET-28a(+)-GFP, pET-28a(+)-sumo, pET-3c-sumo, pET-35b(+), pTXB1, and pCWori.

11. The engineered biological containment system of claim 10, wherein the vector comprises:
a first vector comprising the second nucleic acid and optionally the first nucleic acid;
a second vector comprising the third nucleic acid; and
a third vector comprising the fourth nucleic acid.

12. The engineered biological containment system of claim 10, wherein the vector further comprises a replication origin, a selection marker and a restriction site,
optionally, the replication origin is at least one of ARS and Ori,
optionally, the selection marker is a nucleotide sequence encoding at least one of the following proteins: His, Ura, Leu, Amp and Kan,
optionally, the restriction site is at least one of the following: ApaI, BamHI, BglII, EcoRI, HindIII, KpnI, NcoI, NdeI, NheI, NotI, SacI, SalI, SphI, XbaI and XhoI.

13. An engineered biological containment method, comprising:
introducing a third nucleic acid and a fourth nucleic acid into an organism to be engineered, wherein the third nucleic acid activates the transcription of a first essential gene of the organism to be engineered, and the fourth nucleic acid encodes an unnatural-amino-acid encoding tool; and
including one or more nucleotide sequences encoding first termination codons in a second essential gene of the organism to be engineered and optionally including a nucleotide sequence encoding a second termination codon at the end of a coding region of the second essential gene,
optionally, the first essential gene is the same as or different from the second essential gene.

14. The method of claim 13, wherein the third nucleic acid is an inducible promoter, preferably an inducible promoter pGAL and/or CaMV35S,
optionally, the first termination codon encodes an unnatural amino acid.

15. An engineered organism, comprising an engineered biological containment system of any one of claims 1 to 12,
optionally, the engineered organism comprising an eukaryotic cell and a prokaryote, the eukaryotic cell comprising: a yeast cell, an animal cell comprising a mammalian cell and an insect cell, and a plant cell comprising a tobacco cell, an *Arabidopsis* cell and a *Columnea* cell; the prokaryote comprising *Escherichia coli* and *Bacillus subtilis.*

16. Use of an engineered organism of claim 15 in the production of sustainable biomass comprising food, feed, pharmaceutical, biofuel and material.

17. A method for screening an engineered biological containment system, comprising:
replacing, with a nucleotide sequence encoding a first termination codon, a nucleotide sequence encoding one or more amino acids among the first 20 amino acids at the N-terminus of a protein encoded by a second essential gene of an engineered organism, so as to obtain a mutant library of engineered organisms, wherein the first termination codon encodes an unnatural amino acid;
introducing a fourth nucleic acid encoding an unnatural-amino-acid encoding tool into the mutant library of engineered organisms; and
screening the mutant library of engineered organisms based on a first culture condition, so as to obtain the engineered biological containment system,
optionally, the first culture condition being the presence of the unnatural amino acid.

18. The method of claim 17, further comprising:
introducing a third nucleic acid activating the transcription of a first essential gene of the engineered organism into the mutant library of engineered organisms,
optionally, the third nucleic acid being an inducible promoter, preferably an inducible promoter pGAL and/or CaMV35S,
optionally, the first essential gene being the same as or different from the second essential gene.

19. The method of claim 18, further comprising:
determining a second culture condition according to the inducible promoter; and
screening the mutant library of engineered organisms based on the first culture condition and the second culture condition, so as to obtain the engineered biological containment system,
optionally, the second culture condition being the presence of Gal and Glu.

20. The method of any one of claims 17 to 19, further comprising:
replacing, with the nucleotide sequence encoding the first termination codon, a nucleotide sequence encoding one or more amino acids among the 4th to the 20th amino acids at the N-terminus of the protein encoded by the second essential gene of the engineered organism, so as to obtain a mutant library of engineered organisms.
